# EUROPEAN PATENT SPECIFICATION

(11) **EP 4 541 277 B1**
(45) Date of publication and mention of the grant of the patent: **16.09.2026**
(21) Application number: 24206803.9
(22) Date of filing: 16.10.2024
(51) Int. Cl.: A61B 5/145, A61B 5/1495

(54) **METHOD FOR INITIAL CALIBRATION OF CONTINUOUS BIOMETRICS SENSOR**
VERFAHREN ZUR ANFÄNGLICHEN KALIBRIERUNG EINES KONTINUIERLICHEN BIOMETRISCHEN SENSORS
PROCÉDÉ D'ÉTALONNAGE INITIAL D'UN CAPTEUR BIOMÉTRIQUE CONTINU

(30) Priority: 17.10.2023 KR 20230138531
(43) Date of publication of application: 23.04.2025
(73) Proprietor: I-SENS, INC., Seoul 06646 (KR)
(72) Inventor: CHO, Hee Gyung, 06646 Seoul (KR)
(74) Representative: BCKIP Part mbB

(56) References cited:
- US-A1- 2020 085 356
- US-A1- 2022 160 266
- US-A1- 2023 039 204

## Description

### TECHNICAL FIELD OF THE INVENTION

The present invention relates to a method for initial calibration of a continuous biological sensor. More specifically, the present invention relates a method for initial calibration of a continuous biological sensor, in which generation of initial calibration information based on a wrong initial calibration value may be prevented by generating initial calibration information after whether an initial calibration value obtained by using an additional blood gathering-type sensor for the initial calibration is abnormal is determined and in which based on that the initial calibration value obtained for the initial calibration is abnormal, the initial calibration information may be accurately generated by obtaining an additional initial calibration value to generate the initial calibration information.

### BACKGROUND

Diabetes is a chronic disease that largely occurs among contemporary people. In the Republic of Korea, two million or more people that are 5% of domestic population have diabetes.

Diabetes occurs when an absolutely large amount of a sugar component is in blood because a balance between the blood and sugar is not redressed as insulin formed in pancreas is absolutely or relatively insufficient due to various causes such as obesity, stress, a poor diet, and innate heredity.

Generally, the blood contains a specific concentration of glucose, and a tissue cell gains energy therefrom.

However, when increased more than necessary, the glucose is not appropriately stored in a muscle or a fat cell and is accumulated in the blood. Accordingly, a diabetes patient maintains blood glucose greatly higher than a normal person. As the superabundant blood glucose directly passes through tissues to be discharged in urine, a sugar component absolutely required in each of the tissues of a body becomes insufficient, which causes an abnormality in each of the tissues of the body.

Diabetes has a feature that a subjective symptom thereof is not approximately obvious at an initial stage. As diabetes progresses, unique symptoms of diabetes, such as eating much, drinking much, polyuria, weight loss, general malaise, itching, and hand and foot injuries that are not cured and last long, appear. When diabetes further progresses, complications that develop into visual disturbance, high blood pressure, kidney disease, stroke, periodontal disease, a cramp, neuralgia, gangrene, and the like appear.

In order to diagnose diabetes and manage diabetes to prevent developing the complications, systematic measurement and treatment of the blood glucose may be done simultaneously.

Since diabetes requires constant measurement of blood glucose, demand for a device associated with blood glucose measurement is constantly increased. Various researches show that occurrence of the complications of diabetes is greatly reduced when the diabetes patient strictly controls the blood glucose. Accordingly, regularly measuring the blood glucose is greatly important for the diabetes patient.

In order to control the blood glucose of the diabetes patient, such a blood gathering-type blood glucose meter that uses a finger prick method is mainly used. Such a blood gathering-type blood glucose meter helps control of the blood glucose of the diabetes patient, but accurately identifying a blood glucose value that frequently changes is difficult because the blood gathering-type blood glucose meter shows only a result at a time of measuring. In addition, since the blood gathering-type blood glucose meter always requires blood gathering in order to frequently measure the blood glucose in one day, the diabetes patient has a large burden of blood gathering.

The diabetes patient generally alternates between a hyperglycemia state and a hypoglycemia state. An emergency may occur in the hypoglycemia state. The hypoglycemia state occurs when supply of sugar does not last for a long time, and the diabetes patient may lose consciousness or may die at worst. Thus, immediately discovering the hypoglycemia state is greatly important for the diabetes patient. However, a blood gathering-type blood glucose meter that intermittently measures the blood glucose has a clear limitation.

In order to overcome the limitation on such a blood gathering-type blood glucose meter, a continuous glucose monitoring system (CGMS) that is inserted into a human body to measure the blood glucose at intervals of several minutes has been developed. Control for the diabetes patient and coping with the emergency may be facilitated with the continuous glucose monitoring system.

The continuous glucose monitoring system comprises of a body-attachable unit that is attached to a body region of a user to measure blood glucose, a communication terminal that outputs a received blood glucose value, and the like. The body-attachable unit measure the blood glucose of the user, for example, for fifteen days in a state of being inserted into a body and generates blood glucose information. The body-attachable unit periodically generates the blood glucose information. A blood glucose management application may be disposed in the communication terminal, and the communication terminal may periodically receive the blood glucose information and output the received blood glucose information so that the user may identify the received blood glucose information.

In order to provide accurate blood glucose information to the user, the blood glucose information received from the body-attachable unit may be initially calibrated and then calibrated at intervals of a calibration period in a use period of the body-attachable unit.

In initial calibration, a reference blood glucose value measured through an additional blood glucose meter is used as a calibration value, so that a calibration factor is calculated. A blood glucose value measured by the body-attachable unit is calibrated by using the calibration factor. Afterwards, a new calibration factor is calculated with the reference blood glucose value measured through the blood glucose meter at the intervals of the calibration period in the use period of the body-attachable unit, and the blood glucose value measured by the body-attachable unit is calibrated with the new calibration factor.

When the reference blood glucose value is measured by using an additional blood gathering-type blood glucose meter for calibration, and when the reference blood glucose value is not accurately measured because sugar is put on a finger of the user or when the user inputs a wrong reference blood glucose value by mistake even if the reference blood glucose value is accurately measured, the calibration factor is not accurately calculated. Accordingly, the blood glucose value measured by the body-attachable unit is continuously inaccurately measured.

Particularly, since a sensor of the body-attachable unit may depend on the reference blood glucose value to be initially calibrated at an initial calibration time point after the sensor is inserted into a body, when the wrong reference blood glucose value is input, the blood glucose information on the user may not be accurately measured.

Document US 2023 / 0 039 204 A1 discloses a method for calculating the calibration sensitivity of a sensor for insertion into the body by storing past sensitivities and using at least one of the past sensitivities and a currently calculated sensitivity to calculate a calibration sensitivity of the sensor for insertion into the body. The method calculates the calibration sensitivity by determining whether the reference biometric value used to calculate the calibration sensitivity is within an allowable range.

Document US 2022 / 0 160 266 A1 discloses a method for calibrating a blood glucose value in a continuous blood glucose measurement system. The blood glucose is calibrated by selecting calibration modes on the basis of whether the difference between a blood glucose value measured by a continuous blood glucose measurement system and a reference blood glucose value measured by a separate blood glucose meter is outside a set range. The blood glucose value of a user can be calibrated by activating, to be scrolled on an input window, only the ranges of the blood glucose value and a reference blood glucose value calculated on the basis of a preset critical range, or by forcibly or automatically calibrating the blood glucose value by a second calibration mode which uses multiple reference blood glucose values, when a reference blood glucose value outside the reference blood glucose value range is input.

### SUMMARY OF THE INVENTION

It is an object of the present invention to provide an initial calibration method of determining whether an initial calibration value obtained by using an additional blood gathering-type sensor for initial calibration is abnormal and preventing initial calibration information from being generated based on a wrong initial calibration value.

It is another object of the present invention to provide an initial calibration method of accurately generating initial calibration information by obtaining an additional initial calibration value based on that an initial calibration value obtained for initial calibration is abnormal.

Still another object of the invention is to provide an initial calibration method of accurately generating initial calibration information by using a measured initial calibration value and an additional calibration value based on that the initial calibration value which is obtained for initial calibration and the additional calibration value are abnormal.

To this end, the present invention provides a method for calibration of a blood glucose measurement sensor in accordance with claim 1. There is provided a method for initial calibration of a sensor inserted into a body to measure biological information for a predetermined time, the method including obtaining at least two or more initial calibration values measured at a time point of the initial calibration, determining whether the initial calibration values are abnormal based on the initial calibration values, and generating initial calibration information from the initial calibration values based on whether the initial calibration values are abnormal.

The initial calibration values is obtained by using an additional sensor measuring the biological information at a time of the initial calibration of the sensor.

The initial calibration values may be obtained through an initial calibration interface activated in a user terminal at a time of the initial calibration of the sensor.

Based on that deviations of the initial calibration values exceed a deviation threshold range, the initial calibration values are determined as being abnormal.

Based on that a difference between the initial calibration values exceeds a difference threshold range, the initial calibration values are determined as being abnormal.

Based on that a difference between a first initial calibration value and a second initial calibration value exceeds a difference threshold range, the initial calibration values are determined as being abnormal.

The method further comprises obtaining an additional initial calibration value measured by using an additional sensor based on that the initial calibration values are determined as being abnormal, and determining whether the additional initial calibration value is abnormal, and the initial calibration information is generated by using the additional initial calibration value based on whether the additional initial calibration value is abnormal.

The method may further comprise determining an initial calibration failure based on that the additional initial calibration value is determined as being abnormal, and generating a message showing the initial calibration failure to a user terminal.

The additional initial calibration value may be obtained through an additional calibration interface activated in a user terminal based on that the initial calibration values may be determined as being abnormal.

Based on that a deviation of the additional initial calibration value exceeds the deviation threshold range, the additional initial calibration value is determined as being abnormal.

Based on that a difference between additional initial calibration values comprising the additional initial calibration value exceeds the difference threshold range, the additional initial calibration value is determined as being abnormal.

Based on that a difference between a first additional initial calibration value and a second additional initial calibration value exceeds the difference threshold range, the additional initial calibration value may be determined as being abnormal.

Based on that the additional initial calibration value is determined as being abnormal, the initial calibration information is generated by using the initial calibration values and the additional initial calibration value.

Based on that the additional initial calibration value is determined as being abnormal, the initial calibration information is generated by using two values having a smallest difference among the initial calibration values and the additional initial calibration value.

The initial calibration information may be generated by using two values having a smallest difference among the initial calibration values and the additional initial calibration value which are present between an upper limit threshold value and a lower limit threshold value.

Based on that a difference between one of the initial calibration values and the additional initial calibration value is within the difference threshold range, the additional calibration value may be determined as being normal, and the initial calibration information may be generated by using the additional initial calibration value and a initial calibration value having a difference from the additional calibration value within the difference threshold range among the initial calibration values.

Based on that a difference between additional initial calibration values comprising the additional calibration value, which are consecutively input, is within the difference threshold range, the additional initial calibration values which are consecutively input may be determined as being normal, and the initial calibration information may be generated by using the additional initial calibration values which are consecutively input.

Additional aspects of example embodiments will be set forth in part in the description which follows and, in part, will be apparent from the description, or may be learned by practice of the invention.

According to example embodiments, initial calibration information may be generated after whether an initial calibration value obtained by using an additional blood gathering-type sensor for initial calibration is determined. Through this, the initial calibration information may be prevented from being generated based on a wrong initial calibration value.

According to example embodiments, the initial calibration information may be generated by obtaining an additional initial calibration value based on that the initial calibration value obtained for the initial calibration is abnormal. Through this, the initial calibration information may be accurately generated.

According to example embodiments, the initial calibration information may be generated by using the measured initial calibration value and the additional calibration value based on that the initial calibration value obtained for the initial calibration and the additional calibration value are abnormal. Through this, relatively accurate initial calibration information may be generated while a pain of gathering blood for continuously obtaining a calibration value is reduced.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1 is a schematic diagram illustrating a continuous blood glucose information measurement system according to an example embodiment;
FIG. 2 is a diagram for describing an example of input of an initial calibration value or a periodic calibration value;
FIG. 3 is a function block diagram for describing an initial calibration device of a biological sensor according to the present invention;
FIG. 4 is a function block diagram for describing an example of a calibration controller generating initial calibration information according to the present invention;
FIG. 5 is a diagram for describing an initial calibration method for a biological sensor according to the present invention;
FIG. 6 is a flowchart for describing an example of generating initial calibration information according to the present invention;
FIG. 7 is a flowchart for describing another example of generating initial calibration information according to the present invention;
FIG. 8 is a diagram for describing an example of determining whether an initial calibration value is normal;
FIG. 9 is a diagram for describing an example of generating initial calibration information from an initial calibration value;
FIG. 10 is a diagram for describing another example of determining whether an initial calibration value is normal;
FIG. 11 is a diagram for describing still another example of determining whether an initial calibration value is normal;
FIGS. 12A and 12B are a diagram for describing an example of a user interface for obtaining an initial calibration value in the present invention; and
FIG. 13 is a diagram for describing an example of a notification message showing an initial calibration failure in the present invention.

### DETAILED DESCRIPTION OF EXMPLARY EMBODIMENTS

Technical terms used in the present disclosure are merely to describe predetermined example embodiments. It should be noted that the technical terms is not to limit the present invention. Also, the technical terms used in the present disclosure should be construed, unless the terms are specially defined to have other meanings, as having meanings generally understood by those skilled in the art to which the present invention belong and should not be construed as having excessively inclusive meanings and excessively narrow meanings. In addition, when being wrong technical terms not accurately describing the present invention, the technical terms used in the present disclosure is to be substituted with technical terms understandable by those skilled in the art.

Also, terms in a singular form used in the present disclosure include terms in a plural form unless an apparently and contextually conflicting description is present. In the present disclosure, terms such as "including" or "comprising" should not be construed as that various elements or various operations described in the present disclosure are necessarily included. The terms should be construed as that some of the elements or the operations may not be included or that additional elements or operations may be further included.

Furthermore, the accompanying drawings are merely to easily understand the present invention. It should be noted that the idea of the present invention is not to be construed as being limited by the accompanying drawings.

Hereinafter, a method for initial calibration of a continuous biological sensor according to the present invention will be described in detail with reference to the accompanying drawings.

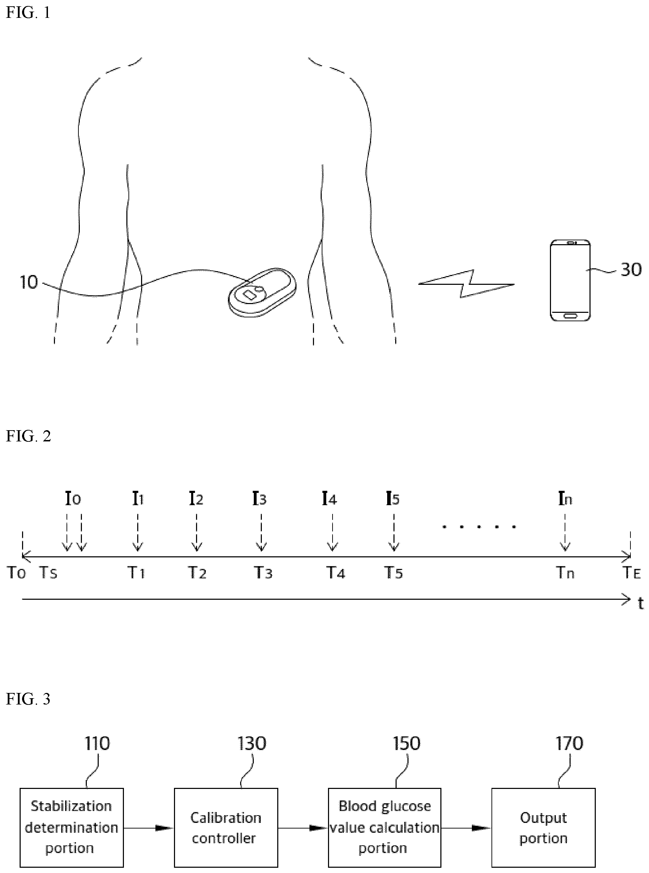

FIG. 1 is a schematic diagram illustrating a continuous blood glucose information measurement system according to an example embodiment.

Hereinafter, a blood glucose value will be described as an example of biological information, and a reference blood glucose value will be described as an example of biological information used as a calibration value. However, a variety of biological information other than the blood glucose value may be measured depending on a field to which the present invention is applied.

Referring to FIG. 1, a continuous biological information measurement system 1 according to an example embodiments of the present invention includes a body-attachable unit 10 and a communication terminal 30.

The body-attachable unit 10 is a device attached to a body to measure the blood glucose value. When the body-attachable unit 10 is attached to the body, an end of a sensor of the body-attachable unit 10 is inserted into skin to measure biological information showing the blood glucose value, namely, blood glucose information from a body fluid of a human body.

The communication terminal 30 is a terminal that may receive the blood glucose information from the body-attachable unit 10, calibrate the received blood glucose information with a calibration factor to change the blood glucose information to calibrated biological information, namely, calibrated blood glucose information, and then display the calibrated blood glucose information to a user. A terminal that may communicate with the body-attachable unit 10, such as a smartphone, a tablet personal computer (PC), a laptop, or the like, may be used as the communication terminal 30. Also, the communication terminal 30 is not limited thereto. Any terminal may be used as long as the terminal includes a communication function and a program or an application may be disposed in the terminal.

In other words, the body-attachable unit 10 generates the blood glucose information which shows a blood glucose value of the user (e.g., an electric current signal) and transmits the blood glucose information to the communication terminal 30, and the communication terminal 30 calibrates the blood glucose information with calibration information, namely, the calibration factor to change the blood glucose information to a calibrated blood glucose value. Depending on a field to which the present invention is applied, the blood glucose information may be directly changed to the blood glucose value in the body-attachable unit 10, and the blood glucose value which is received from the body-attachable unit 10 may be calibrated with the calibration factor in the communication terminal 30.

Hereinafter, the blood glucose information which is measured in the body-attachable unit 10 or the blood glucose value which is changed in the body-attachable unit 10 will be referred to as a measured blood glucose value, and the blood glucose value which is obtained by calibrating the blood glucose information or the changed blood glucose value with the calibration factor in the communication terminal 30 will be referred to as the calibrated blood glucose value.

The body-attachable unit 10 transmits the blood glucose information on the measured blood glucose value to the communication terminal 30 by a request from the communication terminal 30 or periodically at each set time point. For data communication between the body-attachable unit 10 and the communication terminal 30, the body-attachable unit 10 and the communication terminal 30 may be connected in wired communication with each other by a universal serial bus (USB) cable or the like or may be connected in communication with each other with a wireless communication scheme such as infrared communication, near field communication (NFC), or Bluetooth.

When communication is connected between the body-attachable unit 10 and the communication terminal 30, after initial stabilization of the body-attachable unit 10, an initial calibration factor is calculated by using, as an initial calibration value, a reference blood glucose value measured through an additional blood gathering-type sensor (not illustrated), and the measured blood glucose value is calibrated by using the initial calibrated factor. Afterwards, the communication terminal 30 outputs and provides, to the user, the calibrated blood glucose value obtained by calibrating, with the initial calibration factor, the measured blood glucose value received from the body-attachable unit 10.

Desirably, at least two or more initial calibration values may be consecutively obtained at each predetermined time point, and the initial calibration factor may be generated by using the at least two or more obtained initial calibration values.

Further desirably, whether the obtained initial calibration values are abnormal may be determined. Based on that the initial calibration values are determined as being abnormal, an additional initial calibration value may be obtained, and the initial calibration factor may be generated by using the initial calibration values and the additional initial calibration value.

Here, with respect to the initial stabilization, the sensor is determined as being initially stabilized after a predetermined time, for example, after two hours elapse since being inserted into the body. Alternatively, the initial stabilization may be determined based on the measured blood glucose value. The initial stabilization may be a minimum stabilization time or condition required for immediately notifying the user of the calibrated blood glucose value after the sensor is inserted into the body. An additional time, for example, three to ten days may be required for completing stabilization of the sensor.

FIG. 2 is a diagram for describing an example of input of an initial calibration value or a periodic calibration value. Referring to FIG. 2, a body-attachable unit is stabilized from a time point T0 at which the body-attachable unit and a communication terminal are connected in communication to a set stabilization time point TS.

After initial stabilization of the body-attachable unit is completed, an initial calibration value I0 is input. Here, the initial calibration value I0 may be input multiple times in order to accurately calibrate a blood glucose value measured in the body-attachable unit.

After stabilization of the body-attachable unit is completed, new calibration values I1, I2, I3, I4, and the like are input up to a use period expiration time point TE periodically, desirably, at intervals of twelve hours or one day. A calibration factor is calculated from the new calibration values, and a calibrated blood glucose value obtained by calibrating, with the calibration factor, the measured blood glucose value which is received from the body-attachable unit is provided to a user.

FIG. 3 is a function block diagram for describing an initial calibration device of a biological sensor according to the present invention.

In detailed description with reference to FIG. 3, a stabilization determination portion 110 determines whether the sensor is stabilized after the sensor is inserted into skin. Here, the stabilization determination portion 110 may determine that the sensor is stabilized when a set time, for example, two to three hours elapse since the sensor is inserted into the skin or determine that the sensor is stabilized when a blood glucose value measured after the sensor is inserted into the skin converges within a set range.

When the sensor is determined as being stabilized, a calibration controller 130 generates initial calibration information for example, an initial calibration factor by using an initial calibration value measured with an additional blood gathering-type sensor at an initial calibration time point and generates new calibration information, for example, a new calibration factor in a use period of the sensor by using a new calibration value measured with the additional blood gathering-type sensor at a periodic calibration time point.

Here, the initial calibration information or the new calibration information may be a value showing sensitivity of the sensor and may be calculated from a calibration value and a blood glucose value measured by using the sensor to correspond to a time point at which the calibration value is obtained.

A blood glucose value calculation portion 150 calculates a calibrated blood glucose value by calibrating, by using the initial calibration information or the new calibration information, the blood glucose value measured by using the sensor, and an output portion 170 outputs the calculated calibrated blood glucose value to a user.

Desirably, the blood glucose value calculation portion 150 generates, in a blood glucose graph, calibrated blood glucose values which continue over time. The output portion 170 outputs the generated graph to the user.

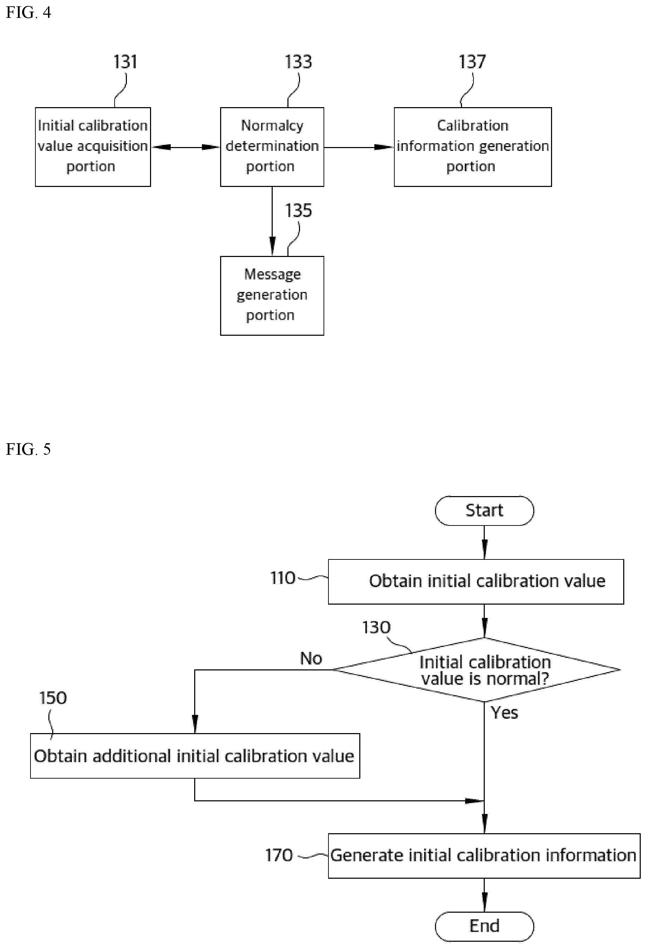

FIG. 4 is a function block diagram for describing an example of a calibration controller generating initial calibration information according to the present invention.

In detailed description with reference to FIG. 4, an initial calibration value acquisition portion 131 obtains an initial calibration value measured with an additional blood gathering-type sensor after the sensor is stabilized. Here, the initial calibration value acquisition portion 131 may activate a user interface for obtaining the initial calibration value, and a user may input the initial calibration value through the user interface.

Desirably, at least two initial calibration values may be obtained. An entry for inputting multiple initial calibration values may be included in the activated user interface.

Desirably, the initial calibration value acquisition portion 131 may activate, in the user interface, a text for guiding an input of the multiple initial calibration values within intervals of a set time. The initial calibration value acquisition portion 131 determines whether the multiple initial calibration values are input within the intervals of the set time. When the multiple initial calibration values are not input within the intervals of the set time, the initial calibration value acquisition portion 131 may additionally activate, in the user interface, a guide text that urges the input of the multiple initial calibration values.

A normalcy determination portion 133 determines whether the multiple initial calibration values are normal based on the multiple initial calibration values which are obtained.

Here, calibration information may be calculated with various scheme. As an example of generating the calibration information, the calibration information may be generated by calculating an average value of the multiple initial calibration values and then dividing the average value by a biological value measured with a sensor of a body-attachable unit. Afterwards, a calibrated blood glucose value of the user may be obtained by multiplying the calibration information and the biological value measured with the sensor of the body-attachable unit together.

As another example of generating the calibration information, final calibration information may be generated by granting, over a time of wearing the sensor of the body-attachable unit, different weighted values to the calibration information generated with the above-described scheme. For example, as the time of wearing the sensor of the body-attachable unit elapses, accuracy of the sensor of the body-attachable unit tends to be increased. Thus, the final calibration information may be generated by granting a weighted value of which a size is gradually decreased as the time of wearing the sensor of the body-attachable unit elapses to the calibration information.

However, Based on that the multiple initial calibration values are determined as being abnormal, the normalcy determination portion 133 performs control to obtain an additional initial calibration value through the initial calibration value acquisition portion 131.

The initial calibration value acquisition portion 131 activates an user interface for obtaining the additional initial calibration value and obtains the additional initial calibration value through the activated user interface. Here, one or more additional initial calibration values may be obtained.

Desirably, an entry for inputting the additional initial calibration value may be included in the user interface, and the initial calibration value acquisition portion 131 may determine whether the additional initial calibration value is input within a set time. When the additional initial calibration value is not input within the set time, the initial calibration value acquisition portion 131 may additionally activate, in the user interface, a guide text that urges an input of the additional initial calibration value.

The normalcy determination portion 133 determines whether the additional initial calibration value is normal based on the obtained additional initial calibration value. Based on that the additional initial calibration value is normal, a calibration information generation portion 137 generates the initial calibration information to be used in initial calibration by using the additional initial calibration value.

In an example embodiment of the present invention, based on that the additional initial calibration value is determined as being normal, a process of obtaining the additional initial calibration value and determining whether the obtained additional initial calibration value is normal may be repeated a set number of times or less.

In another example embodiment of the present invention, based on that the additional initial calibration value is determined as being normal, acquisition of the additional initial calibration value may be stopped in order to reduce a pain of the user which is accompanied by continuously obtaining the additional initial calibration value by using the additional blood gathering-type sensor.

Based on that the additional initial calibration value is determined as being normal, a message generation portion 135 generates a guide message showing an initial calibration failure and outputs the generated message to the user through an output portion.

However, based on that the additional initial calibration value is determined as being abnormal, instead of outputting the guide message to the user, the calibration information generation portion 137 generates auxiliary initial calibration information from an initial calibration value obtained in advance and the additional initial calibration value and calibrates a measured blood glucose value by using the generated auxiliary initial calibration information.

Depending on a field to which the present invention is applied, based on that the additional initial calibration value is determined as being abnormal, while the guide message is output to the user, the measured blood glucose value may be calibrated by generating the auxiliary initial calibration information from the initial calibration value obtained in advance and the additional initial calibration value.

FIG. 5 is a diagram for describing an initial calibration method for a biological sensor according to the present invention.

In detailed description with reference to FIG. 5, after the sensor is inserted into skin and stabilized, an initial calibration value measured with an additional blood gathering-type sensor is obtained through a user interface activated at an initial calibration time point in operation S110. Here, multiple initial calibration values measured with the additional blood gathering-type sensor may be obtained at intervals of a predetermined time or less.

When the multiple initial calibration values are obtained, whether the multiple initial calibration values are normal is determined in operation S 130. Here, with respect to determination of whether the multiple initial calibration values are normal, the multiple initial calibration values are determined as being normal based on that a difference between the multiple initial calibration values is within a difference threshold range, or based on that deviations of the multiple initial calibration values are within a deviation threshold range. However, based on that the difference between the multiple initial calibration values is out of the difference threshold range, or based on that the deviations of the multiple initial calibration values are out of the deviation threshold range, the multiple initial calibration values may be determined as being abnormal.

Based on that the multiple initial calibration values are determined as being normal, in operation S150, initial calibration information is generated from the multiple initial calibration values, and a measured sugar value is calibrated by using the generated initial calibration information.

However, based on that the multiple initial calibration values are determined as being abnormal, an additional initial calibration value is obtained through an additional blood gathering-type sensor in operation S150. In operation S170, whether the obtained additional initial calibration value is normal is determined, and the initial calibration information or auxiliary initial calibration information is generated by using the additional initial calibration value depending on whether the additional initial calibration value is normal.

In a case of periodic calibration in a use period of the sensor, although a wrong calibration value is obtained at a periodic calibration time point, calibration information may be calibrated to some extent based on a blood glucose value obtained up to a previous calibration time point. However, in a case of initial calibration, since a previously obtained blood glucose value is not present, although a wrong initial calibration value is obtained, the initial calibration has a limitation that the initial calibration information is to be generated only based on the initial calibration value. In the present invention, the initial calibration information which is accurate may be generated through a process of obtaining the multiple initial calibration values and determining whether the multiple initial calibration values are normal based on the obtained multiple initial calibration values.

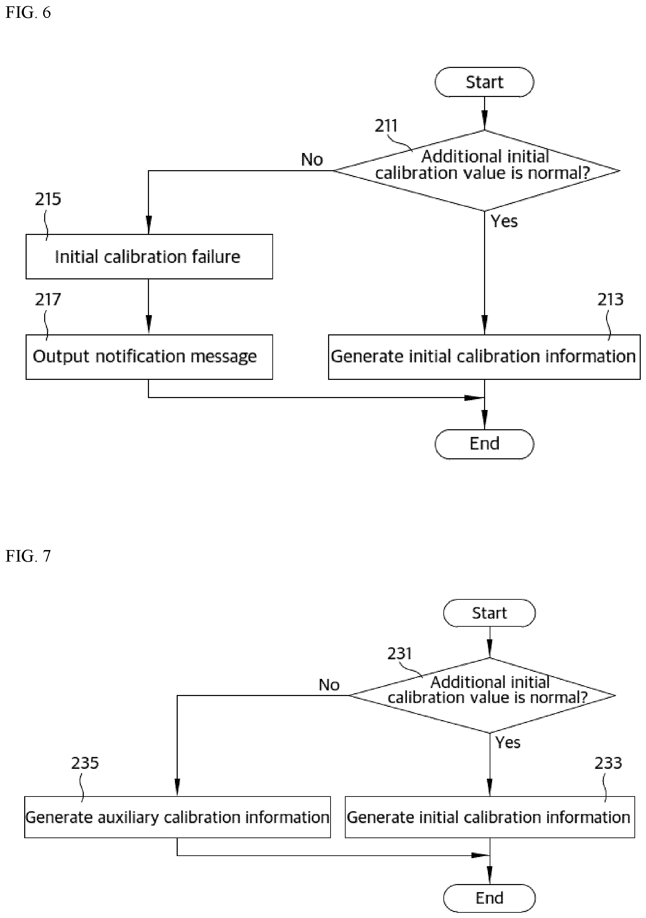

FIG. 6 is a flowchart for describing an example of generating initial calibration information according to the present invention.

In detailed description with reference to FIG. 6, when an additional initial calibration value is obtained, whether the obtained additional calibration value is normal is determined in operation S211.

Based on that the additional initial calibration value is determined as being normal, in operation S213, the initial calibration information is generated based on the additional initial calibration value, and a measured sugar value is calibrated with the generated initial calibration information.

However, based on that the additional initial calibration value is abnormal, initial calibration is determined as a failure in operation S215, and a notification message showing an initial calibration failure is generated and output to a user in operation S217.

FIG. 7 is a flowchart for describing another example of generating initial calibration information according to the present invention.

In detailed description with reference to FIG. 7, when an additional initial calibration value is obtained, whether the obtained additional calibration value is normal is determined in operation S231.

Based on that the additional initial calibration value is normal, the initial calibration information is generated based on the additional initial calibration value in operation S233.

However, based on that the additional initial calibration value is determined as being abnormal, in operation S235, auxiliary initial calibration information is generated by using an obtained initial calibration value and the additional initial calibration value, and a measured sugar value is calibrated by using the generated auxiliary initial calibration information.

Depending on a field to which the present invention is applied, based on that the additional initial calibration value is abnormal, while the auxiliary initial calibration information is generated, a notification message showing that initial calibration has not been normally performed may be generated and output to a user.

As such, even based on that the additional initial calibration value is determined as being normal, a pain accompanied when the user continuously uses an additional blood gathering-type sensor to obtain an initial calibration value may be reduced, and a blood glucose value of the user may be identified by accessorily using the auxiliary initial calibration information until a next periodic calibration time point arrives.

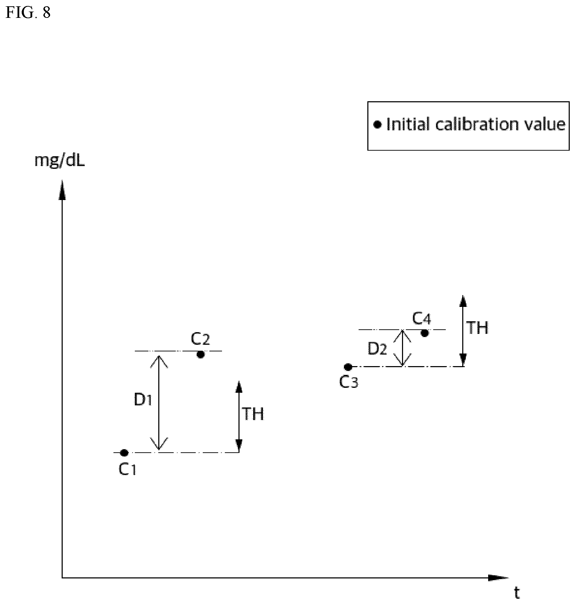

FIG. 8 is a diagram for describing an example of determining whether an initial calibration value is normal.

In detailed description with reference to FIG. 8, when first and second initial calibration values C1 and C2 are consecutively obtained at a time interval, a difference D1 between a first initial calibration value C1 and a second initial calibration value C2 is calculated, and whether the calculated difference D1 is within a difference threshold range TH is determined.

Based on that the first initial calibration value C1 and the second initial calibration value C2 are determined as being abnormal because the difference D1 between the first initial calibration value C1 and the second initial calibration value C2 is out of the difference threshold range TH, first and second additional initial calibration values C3 and C4 are consecutively obtained at a time interval. A difference D2 between a first additional initial calibration value C3 and a second additional initial calibration value C4 is calculated, and whether the calculated difference D2 is within the difference threshold range TH is determined. Based on that the difference D2 between the first additional initial calibration value C3 and the second additional initial calibration value C4 is within the difference threshold range TH, the first additional initial calibration value C3 and the second additional initial calibration value C4 are determined as being normal, and initial calibration information is generated from the first additional initial calibration value C3 and the second additional initial calibration value C4.

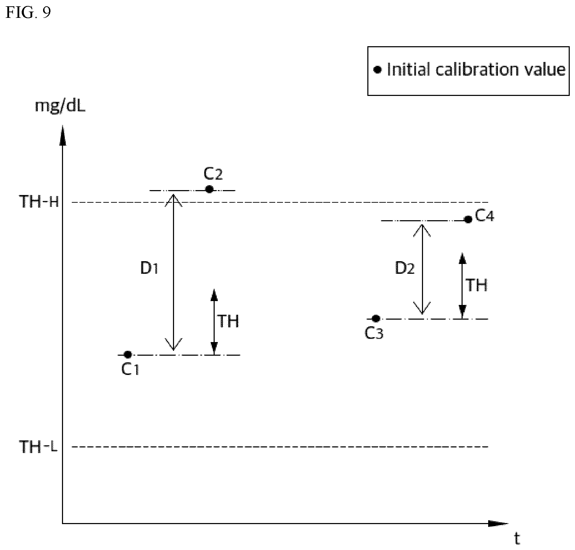

FIG. 9 is a diagram for describing an example of generating initial calibration information from an initial calibration value.

In detailed description with reference to FIG. 9, Based on that the first initial calibration value C1 and the second initial calibration value C2 are determined as being abnormal because the difference D1 between the first initial calibration value C1 and the second initial calibration value C2 is out of the difference threshold range TH, the first additional initial calibration value C3 and the second additional initial calibration value C4 are consecutively obtained at a time interval. Based on that the first additional initial calibration value C3 and the second additional initial calibration value C4 are determined as being abnormal because the difference between D2 the first additional initial calibration value C3 and the second additional initial calibration value C4 is out of the difference threshold range TH, auxiliary initial calibration information may be generated from the first initial calibration value C1, the second initial calibration value C2, the first additional initial calibration value C3, and the second additional initial calibration value C4.

The auxiliary initial calibration information may be generated with two values C2 and C4 having a smallest difference among the first initial calibration value C1, the second initial calibration value C2, the first additional initial calibration value C3, and the second additional initial calibration value C4.

Desirably, the auxiliary initial calibration information may be generated by using two values C1 and C3 having a smallest difference among initial calibration values and additional initial calibration values that are present between an upper limit threshold value TH_H and a lower limit threshold value TH_L except an initial calibration value and an additional initial calibration value that are out of a range between the upper limit threshold value TH_H and the lower limit threshold value TH_L.

Here, the upper limit threshold value TH_H and the lower limit threshold value TH_L may be set to be a maximum value and a minimum value of ordinary blood glucose, respectively. As such, the initial calibration information may be further accurately generated by excluding the initial calibration value or the additional initial calibration value that are out of the range between the upper limit threshold value TH_H and the lower limit threshold value TH_L.

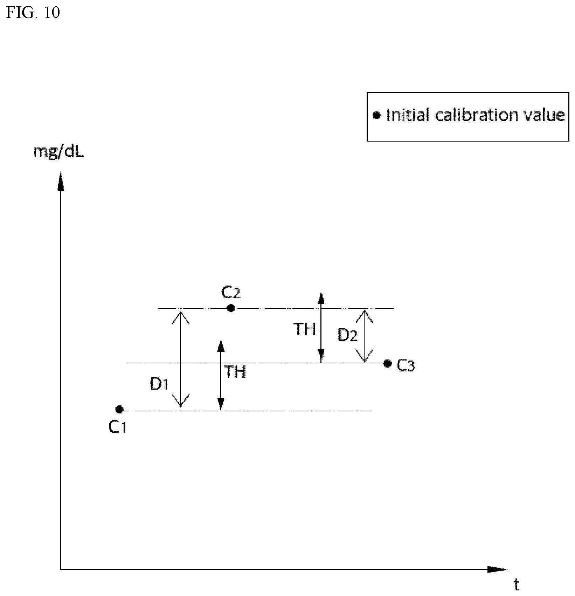

FIG. 10 is a diagram for describing another example of determining whether an initial calibration value is normal.

In detailed description with reference to FIG. 10, when the first and second initial calibration values C1 and C2 are consecutively obtained at a time interval, the difference D1 between the first initial calibration value C1 and the second initial calibration value C2 is calculated, and whether the calculated difference D1 is within the difference threshold range TH is determined.

Based on that the first initial calibration value C1 and the second initial calibration value C2 are determined as being abnormal because the difference D1 between the first initial calibration value C1 and the second initial calibration value C2 is out of the difference threshold range TH, the first additional initial calibration value C3 is obtained.

Based on that a difference D2 between the second initial calibration value C2 and the first additional initial calibration value C3 which is subsequently input is within the difference threshold range TH, the subsequently input first additional initial calibration value C3 is determined as being normal, and initial calibration information is generated by using the second initial calibration value C2 and the first additional initial calibration value C3 which are consecutively input.

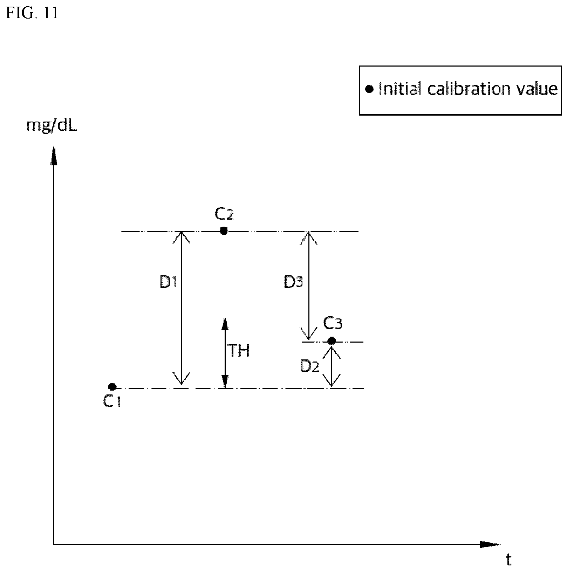

FIG. 11 is a diagram for describing still another example of determining whether an initial calibration value is normal.

In detailed description with reference to FIG. 11, when the first and second initial calibration values C1 and C2 are consecutively obtained at a time interval, the difference D1 between the first initial calibration value C1 and the second initial calibration value C2 is calculated, and whether the calculated difference D1 is within the difference threshold range TH is determined.

Based on that the difference D1 between the first initial calibration value C1 and the second initial calibration value C2 is determined as being abnormal because the difference D1 is out of the difference threshold range TH, the first additional initial calibration value C3 is obtained.

Based on that a difference between the first additional initial calibration value C3 and one of the first initial calibration value C1 and the second initial calibration value C2 is within the difference threshold range TH, the first additional initial calibration value C3 is determined as being normal, and initial calibration information is generated by using the first additional initial calibration value C3 and the first initial calibration value C1 that is one of the initial calibration values C1 and C2 which has a difference from the first additional initial calibration value C3 within the difference threshold range TH.

Based on that a difference between the first additional initial calibration value C3 and each of the first initial calibration value C1 and the second initial calibration value C2 is within the difference threshold range TH, the initial calibration information may be generated by using the first additional initial calibration value C3 and an initial calibration value having a smaller difference.

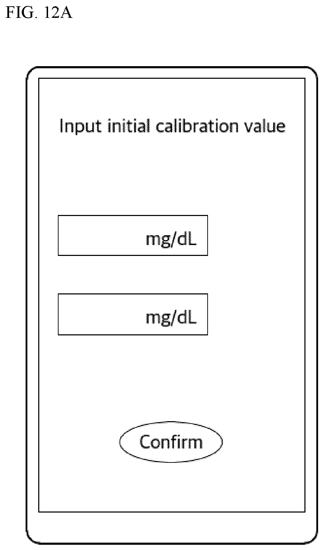

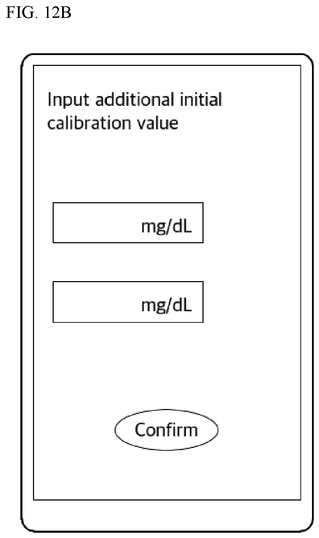

FIGS. 12A and 12B are a diagram for describing an example of a user interface for obtaining an initial calibration value in the present invention.

Referring to FIG. 12A, a first user interface in which an entry for inputting at least two or more initial calibration values is displayed is activated. Multiple calibration values may be input to the first user interface, so that an initial calibration value may be easily and accurately obtained.

Meanwhile, referring to FIG. 12B, based on that the initial calibration value is determined as being abnormal, a second user interface in which an entry for inputting an additional initial calibration value is displayed is activated. Depending on a scheme of determining whether the additional initial calibration value is abnormal, an entry for inputting one additional initial calibration value or inputting two additional initial calibration values is activated in the second user interface. The additional initial calibration value may be input to the first user interface, so that the additional initial calibration value may be easily and accurately obtained.

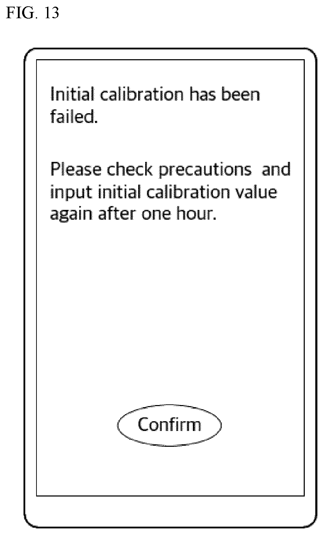

FIG. 13 is a diagram for describing an example of a notification message showing an initial calibration failure in the present invention.

Referring to FIG. 13, when initial calibration based on an initial calibration value and an additional initial calibration value fails, a notification message showing that the initial calibration has failed is output to a user.

The user may recognize, based on the notification message, that the initial calibration has failed and may execute the initial calibration again after a set time elapses.

Desirably, a message showing the initial calibration failure and a message showing that a measured blood glucose value is calibrated by using auxiliary initial calibration information may be simultaneously output to the user.

Depending on a field to which the present invention is applied, the above-described calibration method may be used for not only initial calibration immediately after completion of stabilization after insertion of a sensor of a body-attachable unit into skin but also calibration of the sensor of the body-attachable unit at a periodic calibration time point in a use period of the sensor of the body-attachable unit.

Meanwhile, the above-described example embodiments may be prepared with a computer-executable program and implemented in a general-purpose digital computer for operating the program with a computer-readable recording medium.

The computer-readable recording medium includes a magnetic storage medium (e.g., a read-only memory, a floppy disk, a hard disk, or the like), an optical reading medium (e.g., a compact disc (CD)-ROM, a digital versatile disc (DVD), or the like), and a storage medium such as a carrier wave (e.g., transmission through the Internet).

### LIST OF REFERENCE SIGNS

10: body attachable unit
30: communication terminal
110: stabilization determination portion
130: calibration controller
150: blood glucose value calculation portion
170: output portion
131: initial calibration value acquisition portion
133: normalcy determination portion
135: message generation portion
137: calibration information generation portion

## Claims

1. A method for calibration of a blood glucose measurement sensor, the method comprising:
obtaining (S110) at least two or more calibration values by using an additional sensor measuring biological information at a time of calibrating the sensor;
determining (S130) whether the calibration values are abnormal, wherein, if deviations of the calibration values exceed a deviation threshold range, the calibration values are determined as being abnormal;
if the deviations of the calibration values are determined as being within the deviation threshold range, generating (S170) calibration information from the calibration values,
if the calibration values are determined as being abnormal, obtaining (S150) an additional calibration value,
**characterized by**
determining whether the additional calibration value is abnormal (S211), wherein, if a deviation of the additional calibration value exceeds the deviation threshold range, the additional calibration value is determined as being abnormal (231),
if the additional calibration value is determined as being normal, generating (S213, S233) the calibration information by using the additional calibration value,
if the additional calibration value is determined as being abnormal, generating (S235) auxiliary calibration information by using the calibration values and the additional calibration value, wherein the auxiliary calibration information is generated by using two values having a smallest difference among the calibration values and the additional calibration value.

2. The method of claim 1, wherein the calibration values are inputted through a calibration interface activated in a communication terminal (30) at a time of calibrating the sensor.

3. The method of claim 1 or 2, further comprising:
determining (S215) a calibration failure based on that the additional calibration value is determined as being abnormal; and
outputting (S217) a message showing the calibration failure to a communication terminal(30).

4. The method of any one of any one of claims 1 to 3, wherein the additional calibration value is inputted through an additional calibration interface activated in a communication terminal (30) based on that the calibration values are determined as being abnormal.

5. The method of any one of claims 1 to 4, wherein the additional calibration value comprises a first additional calibration value and a second additional calibration value, and
if a difference between the first additional calibration value and the second additional calibration value exceeds the difference threshold range, the additional calibration value is determined as being abnormal.

6. The method of any one of claims 1 to 5, wherein the auxiliary calibration information is generated by using two values having the smallest difference are present between an upper limit threshold value (TH_H) and a lower limit threshold value (TH_L).

7. The method of any one of claims1 to 6, wherein if a difference between one of the calibration values and the additional calibration value is within a difference threshold range, the additional calibration value is determined as being normal, and
the calibration information is generated by using the additional calibration value and a calibration value having a difference from the additional calibration value within the difference threshold range among the calibration values.

8. The method of any one of claims 1 to 7, wherein if a difference between additional calibration values comprising the additional calibration value, which are consecutively input, is within the difference threshold range, the additional calibration values which are consecutively input are determined as being normal, and
the calibration information is generated by using the additional calibration values which are consecutively input.

9. The method of any one of claims 1 to 8, wherein the calibration values are obtained at an initial calibration time point, and wherein the initial calibration time point is immediately after completion of stabilization of the blood glucose measurement sensor which is inserted into skin.

10. The method of any one of claims 1 to 9, wherein blood glucose information measured from the blood glucose measurement sensor is received at a communication terminal (30), and the communication terminal calibrates the blood glucose information based on the calibration information.

## Patentansprüche

1. Verfahren zur Kalibrierung eines Blutzuckermesssensors, wobei das Verfahren umfasst:
Erhalten (S110) von zumindest zwei oder mehr Kalibrierungswerten unter Verwendung eines zusätzlichen Sensors, der zum Zeitpunkt der Kalibrierung des Sensors biologische Informationen misst;
Bestimmen (S130), ob die Kalibrierungswerte anormal sind, wobei bestimmt wird, dass die Kalibrierungswerte anormal sind, wenn Abweichungen der Kalibrierungswerte einen Abweichungsschwellenbereich überschreiten;
Erzeugen (S170) von Kalibrierungsinformationen aus den Kalibrierungswerten, wenn bestimmt wird, dass die Abweichungen der Kalibrierungswerte innerhalb des Abweichungsschwellenbereichs liegen,
Erhalten (S150) eines zusätzlichen Kalibrierungswerts, wenn bestimmt wird, dass die Kalibrierungswerte anormal sind,
**gekennzeichnet durch**
Bestimmen (S211), ob der zusätzliche Kalibrierungswert anormal ist, wobei bestimmt wird, dass der zusätzliche Kalibrierungswert anormal ist (S231), wenn eine Abweichung der Kalibrierungswerte den Abweichungsschwellenbereich überschreitet;
Erzeugen (S213, S233) der Kalibrierungsinformationen unter Verwendung des zusätzlichen Kalibrierungswerts, wenn bestimmt wird, dass der zusätzliche Kalibrierungswert normal ist,
Erzeugen (S235) von Hilfskalibrierungsinformationen unter Verwendung der Kalibrierungswerte und des zusätzlichen Kalibrierungswerts, wenn bestimmt wird, dass der zusätzliche Kalibrierungswert anormal ist, wobei die Hilfskalibrierungsinformationen unter Verwendung von zwei Werten erzeugt werden, die unter den Kalibrierungswerten und dem zusätzlichen Kalibrierungswert die geringste Differenz aufweisen.

2. Verfahren nach Anspruch 1, wobei die Kalibrierungswerte über eine Kalibrierungsschnittstelle eingegeben werden, die zum Zeitpunkt der Kalibrierung des Sensors in einem Kommunikationsendgerät (30) aktiviert ist.

3. Verfahren nach Anspruch 1 oder 2, ferner umfassend:
Bestimmen (S215) eines Kalibrierungsfehlers, wenn bestimmt wird, dass der zusätzliche Kalibrierungswert anormal ist; und
Ausgeben (S217) einer Nachricht, die den Kalibrierungsfehler anzeigt, an ein Kommunikationsendgerät (30).

4. Verfahren nach einem der Ansprüche 1 bis 3, wobei der zusätzliche Kalibrierungswert über eine zusätzliche Kalibrierungsschnittstelle eingegeben wird, die in einem Kommunikationsendgerät (30) aktiviert wird, wenn bestimmt wird, dass die Kalibrierungswerte anormal sind.

5. Verfahren nach einem der Ansprüche 1 bis 4, wobei der zusätzliche Kalibrierungswert einen ersten zusätzlichen Kalibrierungswert und einen zweiten zusätzlichen Kalibrierungswert umfasst, und
bestimmt wird, dass der zusätzliche Kalibrierungswert anormal ist, wenn eine Differenz zwischen dem ersten zusätzlichen Kalibrierungswert und dem zweiten zusätzlichen Kalibrierungswert den Differenzschwellenbereich überschreitet.

6. Verfahren nach einem der Ansprüche 1 bis 5, wobei die Hilfskalibrierungsinformationen unter Verwendung von zwei Werten erzeugt werden, die die geringste Differenz aufweisen und zwischen einem oberen Grenzschwellenwert (TH_H) und einem unteren Grenzschwellenwert (TH_L) liegen.

7. Verfahren nach einem der Ansprüche 1 bis 6, wobei bestimmt wird, dass der zusätzliche Kalibrierungswert normal ist, wenn eine Differenz zwischen einem der Kalibrierungswerte und dem zusätzlichen Kalibrierungswert innerhalb eines Differenzschwellenbereichs liegt, und
die Kalibrierungsinformationen unter Verwendung des zusätzlichen Kalibrierungswerts und eines Kalibrierungswerts unter den Kalibrierungswerten, dessen Differenz zu dem zusätzlichen Kalibrierungswert innerhalb des Differenzschwellenbereichs liegt, erzeugt werden.

8. Verfahren nach einem der Ansprüche 1 bis 7, wobei bestimmt wird, dass die aufeinanderfolgend eingegebenen zusätzlichen Kalibrierungswerte normal sind, wenn eine Differenz zwischen zusätzlichen Kalibrierungswerten, die den zusätzlichen Kalibrierungswert umfassen und aufeinanderfolgend eingegeben werden, innerhalb des Differenzschwellenbereichs liegt, und
die Kalibrierungsinformationen unter Verwendung der aufeinanderfolgend eingegebenen zusätzlichen Kalibrierungswerte erzeugt werden.

9. Verfahren nach einem der Ansprüche 1 bis 8, wobei die Kalibrierungswerte zum Zeitpunkt der anfänglichen Kalibrierung erhalten werden und wobei der Zeitpunkt der anfänglichen Kalibrierung unmittelbar nach Abschluss der Stabilisierung des in die Haut eingeführten Blutzuckermesssensors liegt.

10. Verfahren nach einem der Ansprüche 1 bis 9, wobei Blutzuckerinformationen, die von dem Blutzuckermesssensor gemessen werden, an einem Kommunikationsendgerät (30) empfangen werden und das Kommunikationsendgerät die Blutzuckerinformationen basierend auf den Kalibrierungsinformationen kalibriert.

## Revendications

1. Procédé d'étalonnage d'un capteur de mesure de glycémie, ledit procédé comprenant :
l'obtention (S110) d'au moins deux valeurs d'étalonnage au moyen d'un capteur additionnel mesurant des informations biologiques au moment de l'étalonnage du capteur ;
la détermination (S130) si les valeurs d'étalonnage sont anormales, où, si des écarts des valeurs d'étalonnage dépassent une plage d'écart seuil, les valeurs d'étalonnage sont déterminées anormales ;
si les écarts des valeurs d'étalonnage sont déterminés compris dans la plage d'écart seuil, la génération (S170) d'informations d'étalonnage à partir des valeurs d'étalonnage,
si les valeurs d'étalonnage sont déterminées anormales, l'obtention (S150) d'une valeur d'étalonnage complémentaire,
**caractérisé par**
une détermination si la valeur d'étalonnage complémentaire est anormale (S211), où, si un écart de la valeur d'étalonnage complémentaire dépasse la plage d'écart seuil, la valeur d'étalonnage complémentaire est déterminée anormale (231),
si la valeur d'étalonnage complémentaire est déterminée normale, la génération (S213, S233) des informations d'étalonnage au moyen de la valeur d'étalonnage complémentaire,
si la valeur d'étalonnage complémentaire est déterminée anormale, la génération (S235) d'informations d'étalonnage auxiliaires au moyen des valeurs d'étalonnage et de la valeur d'étalonnage complémentaire, les informations d'étalonnage auxiliaires étant générées au moyen de deux valeurs ayant une différence minimale entre les valeurs d'étalonnage et la valeur d'étalonnage complémentaire.

2. Procédé de la revendication 1, où les valeurs d'étalonnage sont entrées par une interface d'étalonnage activée dans un terminal de communication (30) au moment de l'étalonnage du capteur.

3. Procédé selon la revendication 1 ou la revendication 2, comprenant en outre :
la détermination (S215) d'un étalonnage défectueux sur la base d'une détermination d'anomalie de la valeur d'étalonnage complémentaire ; et
la transmission (S217) d'un message d'étalonnage défectueux à un terminal de communication (30).

4. Procédé selon l'une des revendications 1 à 3, où la valeur d'étalonnage complémentaire est entrée par une interface d'étalonnage complémentaire activée dans un terminal de communication (30) sur la base d'une détermination d'anomalie des valeurs d'étalonnage.

5. Procédé selon l'une des revendications 1 à 4, où la valeur d'étalonnage complémentaire comprend une première valeur d'étalonnage complémentaire et une deuxième valeur d'étalonnage complémentaire, et où,
si une différence entre la première valeur d'étalonnage complémentaire et la deuxième valeur d'étalonnage complémentaire dépasse la plage seuil de différence, la valeur d'étalonnage complémentaire est déterminée anormale.

6. Procédé selon l'une des revendications 1 à 5, où les informations d'étalonnage auxiliaires sont générées au moyen de deux valeurs présentant une différence minimale entre une valeur seuil limite supérieure (TH_H) et une valeur seuil limite inférieure (TH_L).

7. Procédé selon l'une des revendications 1 à 6, où, si une différence entre une des valeurs d'étalonnage et la valeur d'étalonnage complémentaire est comprise dans une plage seuil de différence, la valeur d'étalonnage complémentaire est déterminée normale, et
les informations d'étalonnage sont générées au moyen de la valeur d'étalonnage complémentaire et d'une valeur d'étalonnage présentant une différence par rapport à la valeur d'étalonnage complémentaire dans la plage seuil de différence parmi les valeurs d'étalonnage.

8. Procédé selon l'une des revendications 1 à 7, où, si une différence entre des valeurs d'étalonnage complémentaires comprenant la valeur d'étalonnage complémentaire, entrées consécutivement, est comprise dans la plage seuil de différence, les valeurs d'étalonnage complémentaires entrées consécutivement sont déterminées normales, et
les informations d'étalonnage sont générées au moyen des valeurs d'étalonnage complémentaires entrées consécutivement.

9. Procédé selon l'une des revendications 1 à 8, où les valeurs d'étalonnage sont obtenues à un moment d'étalonnage initial, et où le moment d'étalonnage initial suit immédiatement la fin de la stabilisation du capteur de mesure de glycémie inséré dans la peau.

10. Procédé selon l'une des revendications 1 à 9, où les informations de glycémie mesurées par le capteur de glycémie sont reçues sur un terminal de communication (30), et où le terminal de communication étalonne les informations de glycémie sur la base des informations d'étalonnage.
